# EUROPÄISCHE PATENTSCHRIFT

(11) **EP 2 806 981 B1**
(45) Veröffentlichungstag und Bekanntmachung des Hinweises auf die Patenterteilung: **17.08.2016**
(21) Anmeldenummer: 12810145.8
(22) Anmeldetag: 19.01.2013
(51) Int. Cl.: B05D 7/22, B05D 7/08, B05B 13/04, B05B 13/06, B05D 1/02

(54) **VERFAHREN ZUR BESCHICHTUNG EINES STENTS**
METHOD FOR COATING A STENT
PROCÉDÉ POUR REVÊTIR UNE ENDOPROTHÈSE

(30) Priorität: 23.01.2012 US 201261589409 P
(43) Veröffentlichungstag der Anmeldung: 03.12.2014
(73) Patentinhaber: Cortronik GmbH, 18119 Rostock-Warnemünde (DE)
(72) Erfinder: STERNBERG, Katrin, 18057 Rostock (DE); KROEMER, Heyo K., 17498Neuenkirchen (DE); SCHMITZ, Klaus-Peter, 18119 Warnemünde (DE); WEITSCHIES, Werner, 17498 Neuenkirchen (DE); GRABOW, Niels, 18055 Rostock (DE); HARDER, Claus, 91080 Uttenreuth (DE); LITTWIN, Peter, 18109 Rostock (DE); BAJER, Dalibor, 18147 Rostock (DE)
(74) Vertreter: Galander, Marcus
(86) Internationale Anmeldenummer: PCT/EP2012/074818
(87) Internationale Veröffentlichungsnummer: WO 2013/110393

(56) Entgegenhaltungen:
- US-A1- 2005 238 829

## Beschreibung

Die Erfindung betrifft ein Beschichtungsverfahren, das mithilfe einer Vorrichtung durchgeführt wird.

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, zum Beispiel Stents), zur Befestigung und temporären Fixierung von Gewebeimplantaten und Gewebstransplantationen, aber auch zu orthopädischen Zwecken, zum Beispiel als Nagel, Platte oder Schraube.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die vorrangig zur Abdichtung des Aneuryrismas dienen. Die Stützfunktion ist zusätzlich gegeben.

Stents besitzen eine Umfangswandung von ausreichender Tragkraft, um das verengte Gefäß im gewünschten Maße offen zu halten und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel bis zur Freisetzung des Implantats durch einen Schutzschlauch auf das Material ausgeübt.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatwerkstoffs in verschiedener Weise. Entsprechend der Reaktion des Biosystems können die Implantatwerkstoffe in bioaktive, bioinerte und degradierbare, beispielsweise resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (zum Beispiel cp Titan, TiAl6V4 oder Ti-Al6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Stents mit einer Beschichtung zur lokalen Wirkstofffreisetzung (sogenannte Local Drug Delivery (LDD)-Systeme) sind seit langem bekannt und werden in der Praxis vielfältig verwendet. Durch die Medikation soll einer Restenose vorgebeugt und in manchen Fällen zusätzlich der Einheilprozess unterstützt werden. Dabei ist es günstig, den antiproliferativen Wirkstoff zur Verhinderung der Restenose im wesentlichen nur abluminal bereit zu stellen, da er dann hauptsächlich in die Gefäßwand eluiert wird, wo er zur Wirkung kommen soll. Auf diese Weise wird angestrebt, eine Verzögerung der Einheilung auf der luminalen Seite des Stents durch den antiproliferativen Wirkstoff weitgehend zu vermeiden. Der Prozeß der Einheilung kann zusätzlich durch einen entsprechenden luminal beschichteten zweiten Wirkstoff gefördert werden. Die Kombination einer luminalen und einer abluminalen Beschichtung hat ferner den Vorteil, daß eine formschlüssige Beschichtung erreicht werden kann, was die mechanische Stabilität der Beschichtung deutlich erhöht.

Teilweise erfolgt eine lokale Differenzierung der Wirkstofffreisetzung mit Hilfe von Wirkstoffdepots, die auf der Oberfläche des Stents angeordnet werden. Es sind ferner Stentvarianten bekannt, bei denen lediglich eine rein abluminale Beschichtung aus Polymer und Wirkstoff aufgetragen wird. Darüber hinaus wurden Systeme beschrieben, bei denen abluminal und luminal verschiedene Wirkstoffe aus Polymerbeschichtungen freigesetzt werden. WO 2010/120552 A1 beschreibt Stents, die eine luminal und abluminal divergierende Beschichtungsdicke aufweisen. Auch aus anderen Gründen werden Stents beschichtet, beispielsweise um das Korrosionsverhalten von biokorrodierbaren Implantatwerkstoffen zu beeinflussen.

Für die Beschichtung von Stents mit Wirkstoffen und anderen Materialien, wie zum Beispiel als Trägermatrix oder Korrosionsschutz dienenden Polymeren, ist eine Reihe von Verfahren industriell etabliert. Hierzu zählen Sprühzerstäubung auf Basis von Trägergasen, Ultraschallzerstäubung, Rotationszerstäubung, Rollwalzen und Verfahren auf Basis des Tintenstrahldruckes.

In US 2008/0226812 A1 wird ein Beschichtungsapparat beschrieben, der sowohl luminal (Innenseite des Stents) als auch abluminal (Außenseite des Stents) beschichten kann. Dies geschieht mit Hilfe der Technologie eines Tintenstrahldruckers, wobei der Stent während der Beschichtung gedreht wird. Mit dem Verfahren können auch Beschichtungen, die aus mehreren Schichten bestehen, aufgebracht werden.

In US 2007/0288088 A1 wird die Beschichtung eines Stents durch eine Sprühbeschichtung beschrieben, bei der der Stent sich dreht und die Düse sich im Abstand von 6,5 mm zum Stent befindet.

US 2011/0073036 A1 beschreibt eine Vorrichtung zur luminalen Beschichtung eines Stents. Die Vorrichtung umfasst eine Hülse, die den Stent während der Beschichtung aufnimmt, wobei der Stent schlüssig an der Innenseite der Hülse anliegt. Über einen beweglich gelagerten Sprühdorn, der in die mit dem Stent beschickte Hülse einfährt, erfolgt eine Sprühbeschichtung auf der luminalen Seite des Stents.

US 2005/0238829 A1 beschreibt eine Vorrichtung und ein Verfahren zur Sprühbeschichtung eines Stents.

Die Beschichtungsverfahren und Apparaturen des Standes der Technik weisen den Nachteil auf, dass eine differenzierte Auftragung der Beschichtung auf der luminalen und abluminalen Seite des Stents nicht möglich oder erschwert ist. Zudem haben separat aufgetragene luminale oder abluminale Beschichtungen häufig eine geringe Stabilität, weshalb sie sich unkontrolliert vom Implantat ablösen können.

Eine bekannte Vorrichtung welche zur Beschichtung eines Stents verwendet werden kann, umfasst eine Halterung für den Stent, eine Sprüheinrichtung mit einem Sprühdorn und eine Luftdüse. Der Sprühdorn, die Luftdüse und die Halterung sind so ausgelegt und zueinander angeordnet, dass der Sprühdorn während der Beschichtung von einer Seite in den Stent ragt und die Luftdüse von der gegenüberliegenden Seite in den Stent ragt.

Ein weiterer Aspekt der Erfindung liegt in der Bereitstellung eines Verfahrens zur Beschichtung eines Stents, umfassend die Schritte:
a) Bereitstellen der zuvor beschriebenen Vorrichtung zur Beschichtung des Stents;
b) Luminale Beschichtung des Stents durch Einleiten eines Sprühstrahls über den Sprühdorn sowie eines Luftstroms über die Luftdüse, derart, dass der Sprühstrahl durch den Luftstrom im Gegenstrombereich radial nach Außen abgelenkt wird; und
c) Verschieben einer Lage des Gegenstrombereichs zum Stent durch wenigstens eine der Maßnahmen ausgewählt aus Bewegung des Stents, Bewegung des Sprühdorns, Bewegung der Luftdüse, Regulierung des Sprühstrahls und Regulierung des Luftstroms.

Der Erfindung liegt die Erkenntnis zugrunde, dass eine luminale und/oder abluminale Beschichtung in sehr einfacher und zuverlässiger Weise über ein Sprühverfahren erreicht werden kann, bei dem mittels einer Luftdüse ein Gegenstrom erzeugt wird. Dazu weist die Vorrichtung eine Sprüheinrichtung auf mit einem Sprühdorn, dessen Außendurchmesser geringer als der Innendurchmesser der Stents ist. Ferner ist eine Luftdüse vorhanden, deren Außendurchmesser ebenfalls geringer ist als der Innendurchmesser des Stents. Der Stent wird während des Beschichtungsverfahrens von einer Halterung aufgenommen, derart dass die seitlichen Öffnungen des Stents zugänglich sind und die Seitenwandungen nicht abgeschirmt werden. Der Sprühdorn wird nun von der einen Seite und die Luftdüse von der gegenüberliegenden Seite in den Stent eingefahren. Während des Beschichtungsverfahrens wird dann mittels des Sprühdorns ein Sprühstrahl erzeugt und über die Luftdüse ein Luftstrom auf diesen Sprühstrahl gerichtet. In dem Bereich, in dem Sprühstrahl und Luftstrom aufeinander treffen (hier als Gegenstrombereich bezeichnet) wird der Sprühstrahl radial nach außen abgelenkt und beschichtet somit in diesem Bereich die luminale Oberfläche des Stents. Durch unterschiedliche Maßnahmen ist es nun möglich, den Gegenstrombereich relativ zur Lage des Stents zu verändern, so dass eine lokal differenzierte luminale Beschichtung des Stents möglicht ist. Diese Maßnahmen umfassen eine Bewegung von Stent, Sprühdorn, oder Luftdüse sowie die Regulierung von Sprühstrahl und Luftstrom. Das erfindungsgemäße Beschichtungsverfahren führt zu einer formschlüssigen, mechanisch sehr stabilen Beschichtung.

Vorzugsweise ist die Vorrichtung derart ausgelegt, dass während der Beschichtung die Lage des Stents zum Sprühdorn und zur Luftdüse veränderbar ist. Insbesondere ist die Halterung derart ausgelegt, dass der Stent während der Beschichtung in Richtung des Sprühdorns oder in Richtung der Luftdüse bewegbar ist. Nach dieser Variante wird mit anderen Worten der Stent entlang einer von Sprühdorn und Luftdüse gebildeten Achse mittels der Halterung während des Beschichtungsvorgangs bewegt. Die Einstellung von Lage und Strömungsgeschwindigkeit im Gegenstrombereich ist in diesem Bereich besonders einfach experimentell zu ermitteln und vereinfacht die Implementierung des Verfahrens in den industriellen Fertigungsprozess.

Ferner weist die Sprüheinrichtung eine Sprühdüse auf, die auf eine abluminale Seite des Stents gerichtet ist. Nach einer dazugehörigen Variante des Verfahrens wird im Schritt a) eine Vorrichtung zur Beschichtung des Stents bereitgestellt, bei der die Sprüheinrichtung eine solche Sprühdüse aufweist, die auf eine abluminale Seite des Stents gerichtet ist. Die abluminale Beschichtung kann zeitgleich mit der oben beschriebenen luminalen Beschichtung erfolgen. Zur zeitgleichen abluminalen Beschichtung des Stents wird während der Durchführung der Schritte b) und c) ein Sprühstrahl der Sprühdüse auf den Gegenstrombereich gerichtet. Die abluminale Beschichtung kann aber auch vor oder nach der luminalen Beschichtung erfolgen, wobei ein Sprühstrahl auf die Außenseite des Stents gerichtet wird. Dabei kann die Menge des unabsichtlich auf die luminale Oberfläche gelangenden Beschichtungsmaterials optional durch einen Luftstrom aus der Luftdüse oder auch einen Gegenstrombereich, der mittels Luftdüse und ohne Wirkstoff betriebenem Sprühdorn erzeugt wird, reduziert werden.

Entsprechend weist die Sprüheinrichtung der Vorrichtung eine zusätzliche Sprühdüse auf, die auf die Außenseite des Stents gerichtet ist. Durch die im Gegenstrombereich radial nach außen hin gerichtet Strömung kann im Zuge der abluminalen Beschichtung weitgehend verhindert werden, dass Beschichtungsmaterial für die abluminale Beschichtung in das Innere des Stents eindringen kann. Alternativ oder ergänzend wird mittels des Sprühdorns oder der Luftdüse ein Überdruck im Innern des Stents erzeugt, der ein Eindringen des von der Sprühdüse erzeugten Sprühstrahls verhindern soll oder eindringendes Material seitlich austrägt.

Während der abluminalen Beschichtung muss der von dem Sprühdorn erzeugte Sprühstrahl kein Trägermaterial oder Wirkstoff enthalten, es sei denn eine geringe Vermischung für die abluminale Beschichtung ist erwünscht. Die Zusammensetzung der Beschichtungen auf der luminalen und abluminalen Seite der Stents sind in der Regel verschieden, das heißt sie bestehen aus verschiedenen Trägermaterialien und/oder verschiedenen Wirkstoffen. So werden vorzugsweise wachstumshemmende Wirkstoffe auf der abluminalen Oberseite des Stents deponiert, während auf der luminalen Oberfläche einheilungsfördernde Substanzen aufgebracht werden. Vorzugsweise wird also luminal und abluminal mit unterschiedlichen Wirkstoffen beschichtet. Insbesondere werden luminal Atorvastatin und abluminal Sirolimus aufgetragen.

Die Wirkstoffe können insbesondere in eine Trägermatrix eingebettet werden. Die Trägermatrix kann biokorrodierbar sein und besteht vorzugsweise aus PLLA.

Ein weiterer Aspekt der Erfindung betrifft einen Stent, der nach dem zuvor beschriebenen Verfahren hergestellt wird.
- Fig. 1: Eine schematische Darstellung der Vorrichtung zur luminalen Beschichtung des Stents
- Fig. 2: Eine schematische Darstellung der Vorrichtung zur abluminalen Beschichtung des Stents
- Fig. 3: Schematische Darstellung eines transversal geschnittenen Implantatquerschnitts mit luminaler und abluminaler Schichtdicke nach sequentieller Kombination einer separaten Innen- und Außenbeschichtung
- Fig. 4: Schichtdickenauswertung für die Außenbeschichtung von Implantatquerschnitten in vier transversalen Schnittebenen zur Untersuchung der longitudinalen Schichtdickenkonstanz
- Fig. 5: Schichtdickenauswertung für die Innenbeschichtung von Implantatquerschnitten in vier transversalen Schnittebenen zur Untersuchung der longitudinalen Schichtdickenkonstanz

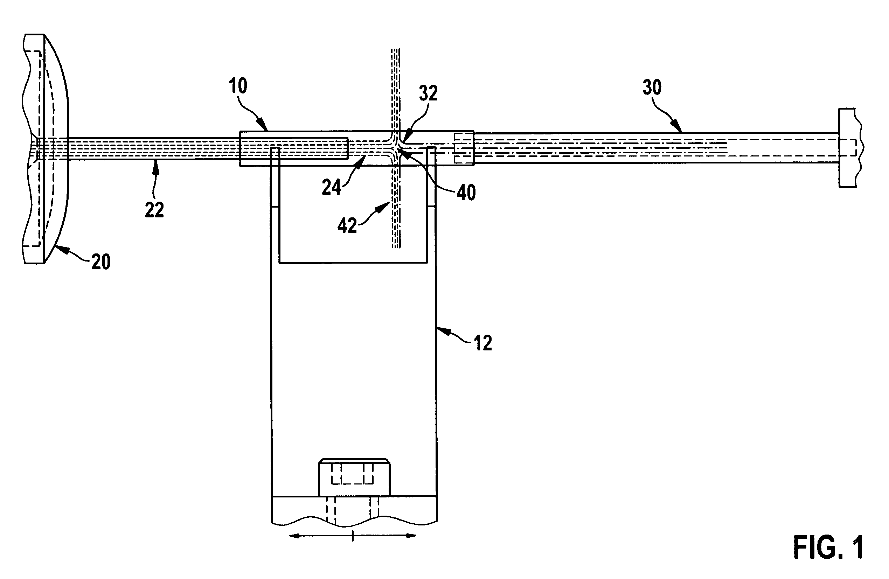

Figur 1 illustriert schematisch das Prinzip der luminalen Beschichtung eines Stents 10 mithilfe der Vorrichtung. Die Vorrichtung umfasst dazu eine Halterung 12, die als Aufnahme für den Stent 10 während des Beschichtungsvorganges dient. Die Halterung 12 ist mittels hier nicht näher dargestellter Aktuatoren zur translatorischen Bewegung ausgelegt.

Die Vorrichtung umfasst ferner eine Sprüheinrichtung 20 mit einem Sprühdorn 22. Mittels der Sprüheinrichtung 20 wird ein Sprühstrahl 24 aus dem Beschichtungsmaterial und einem geeigneten Träger erzeugt. Der Sprühstrahl 24 wird über den Sprühdorn 22 in das Innere des Stents 10 geleitet.

An der gegenüberliegenden Seite des Stents sitzt eine Luftdüse 30, die einen Luftstrom 32 erzeugt.

Der Sprühstrahl 24 und der Luftstrom 32 treffen in einem Gegenstrombereich 40 aufeinander und entsprechend wird der Sprühstrahl 24 - nunmehr vermischt mit dem Luftstrom 32 - radial nach außen abgelenkt und trifft dort auf die zu beschichtende luminale Oberfläche des Stents 10. Teilweise tritt dieser radial nach außen gerichtete Mischstrom 42 auch durch die Öffnungen der filigranen Stützstruktur des Stents 10.

Um den Ort der luminalen Beschichtung zu verändern, wird die Halterung 12 entlang der von Sprühdorn 22 und Luftdüse 30 gebildeten Achse bewegt. Entsprechend verändert sich die relative Lage des Gegenstrombereiches 40 zum Stent 10. Die Zusammensetzung der luminalen Beschichtung in einem bestimmten luminalen Abschnitt des Stents 10 kann durch die Veränderung der Zusammensetzung des Sprühstrahls 24 angepasst werden. Die Schichtdicke lässt sich an jedem beliebigen umlaufenden Abschnitt der luminalen Oberfläche des Stents 10 durch die Dauer der Beschichtung und die Beschichtungszusammensetzung beeinflussen.

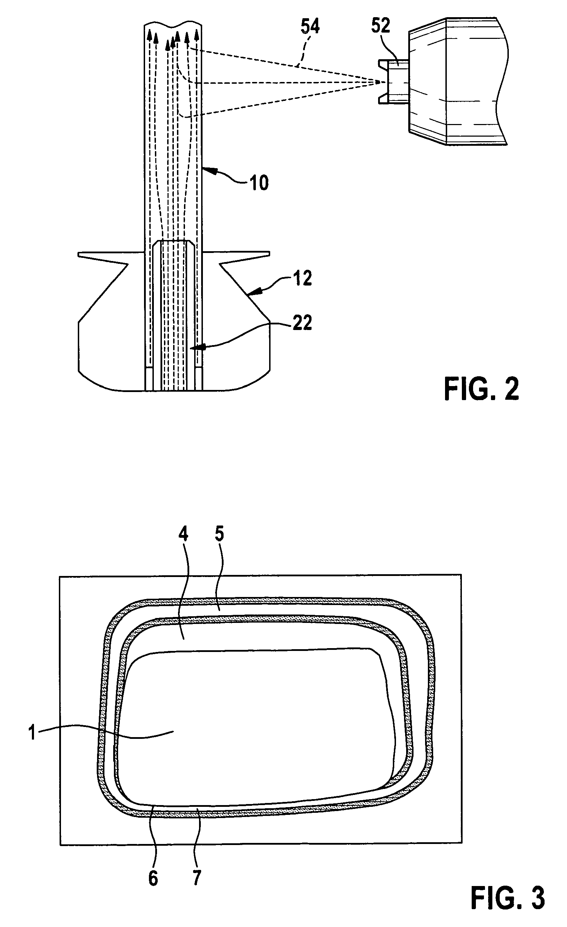

Figur 2 dient der Verdeutlichung einer Variante der Beschichtungsvorrichtung, bei der die Sprüheinrichtung eine zusätzliche Sprühdüse 52 aufweist, die zur Außenbeschichtung des Stents 10 dient. Über die Sprühdüse 52 wird ein Sprühstrahl 54 während der abluminalen Beschichtung auf den Stent 10 geleitet. Im Inneren des Stents 10 kann mittels des Sprühdorns 22 und der hier nicht dargestellten Luftdüse 30 ein Überdruck erzeugt werden, der einen durch die filigranen Strukturen des Stents 10 radial nach außen gerichteten Luftstrom bedingt. Dieser Luftstrom wird so eingestellt, dass er ein Eindringen des Sprühstrahls 54 verhindert, aber eine Beschichtung der abluminalen Oberfläche des Stents 10 noch ermöglicht wird. Ebenso kann die unerwünschte Deposition des abluminalen Schichtmaterials auf der luminalen Oberfläche durch einen mittels Luftdüse oder Sprühdorn erzeugten Luftstrom reduziert werden.

### Ausführungsbeispiel

Ein Stent wird mittels der vorab beschriebenen Vorrichtung zunächst auf der luminalen Seite mit einer Beschichtung aus Atorvastatin in PLLA und anschließend von seiner abluminalen Seite mit einer Beschichtung von Sirolimus in PLLA versehen. Die Beschichtungsdicke auf der Innenseite des Stents solle dabei etwa das zwei- bis dreifache der Beschichtungsdicke der Außenseite betragen.

Figur 3 zeigt eine schematische Darstellung eines transversal geschnittenen Implantatquerschnitts mit luminaler und abluminaler Schichtdicke nach Durchführung der sequentiellen Kombination einer separaten Innen- und Außenbeschichtung.

In der Mitte der Darstellungen findet man den Querschnitt durch einen Stent-Strut (1). Die Oberfläche des Struts ist mit einer inneren Schicht (4, 6) umgeben, wobei die luminale Schichtdicke (4) deutlich größer ist, als die abluminale (6). Dennoch ist die abluminale Schichtdicke (6) größer als Null, so dass sich für die innere Schicht (4, 6) sowohl eine deutliche Anreicherung an der luminalen Oberfläche, als auch ein die mechanische Stabilität der inneren Schicht erhöhender Formschluß ergibt. Die äußere Schicht (5, 7) zeigt ebenfalls einen Formschluß, wobei hier die abluminale Schichtdicke (7) etwas geringer als die luminale (5) ist.

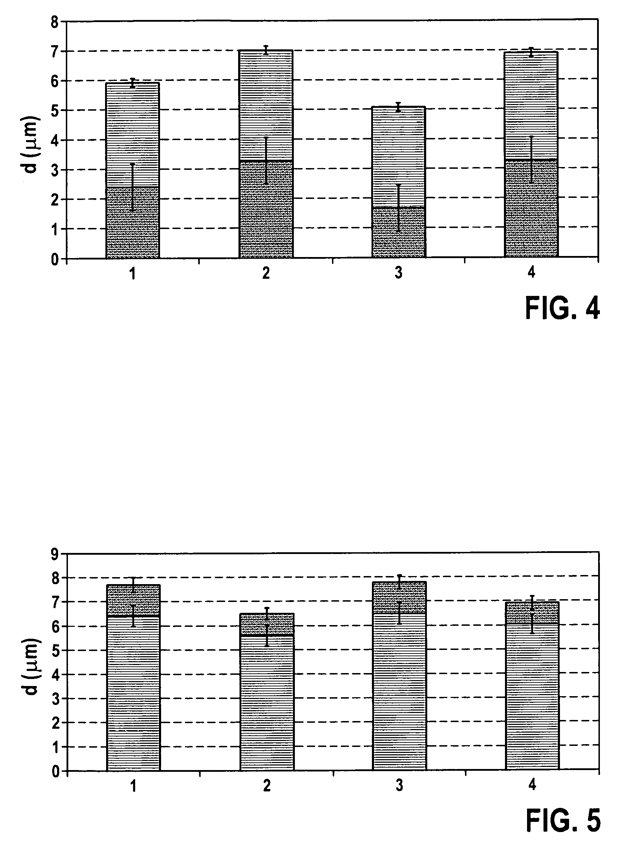

Figur 4 zeigt eine Schichtdickenauswertung für eine Außenbeschichtung und Figur 5 zeigt eine Schichtdickenauswertung für eine Innenbeschichtung von Implantatquerschnitten in vier transversalen Schnittebenen zur Untersuchung der longitudinalen Schichtdickenkonstanz und der luminalen/abluminalen Beschichtungsselektivität. In Figur 4 hellgrau dargestellt ist die Schichtdicke des auf der abluminalen Implantatoberfläche abgeschiedenen Schichtmaterials, dunkelgrau dargestellt ist die Schichtdicke des auf der luminalen Implantatoberfläche abgeschiedenen Schichtmaterials. In Figur 5 hellgrau dargestellt ist die Schichtdicke des auf der luminalen Implantatoberfläche abgeschiedenen Schichtmaterials, dunkelgrau dargestellt ist die Schichtdicke des auf der abluminalen Implantatoberfläche abgeschiedenen Schichtmaterials.

Das Ausführungsbeispiel ist lediglich eine spezielle Ausführungsform der Erfindung und stellt keine Einschränkung der Allgemeinheit des Schutzumfangs dar.

## Patentansprüche

1. Verfahren zur Beschichtung eines Stents, umfassend die Schritte:
a) Bereitstellen einer Vorrichtung zur Beschichtung des Stents, die eine Halterung für den Stent, eine Sprüheinrichtung mit einem Sprühdorn und eine Luftdüse umfasst, wobei der Sprühdorn, die Luftdüse und die Halterung so ausgelegt und zueinander angeordnet sind, dass der Sprühdorn während der Beschichtung von einer Seite in den Stent ragt und die Luftdüse von der gegenüberliegenden Seite in den Stent ragt, und wobei während der Beschichtung eine Lage des Stents zum Sprühdorn und zur Luftdüse veränderbar ist oder der Stent während der Beschichtung in Richtung des Sprühdorns oder in Richtung der Luftdüse bewegbar ist, und wobei
die Sprüheinrichtung eine Sprühdüse aufweist, die auf eine abluminale Seite des Stents gerichtet ist,
b) Luminale Beschichtung des Stents durch Einleiten eines Sprühstrahls über den Sprühdorn sowie eines Luftstroms über die Luftdüse, derart, dass der Sprühstrahl durch den Luftstrom im Gegenstrombereich radial nach Außen abgelenkt wird; und
c) Verschieben einer Lage des Gegenstrombereichs zum Stent durch wenigstens eine der Maßnahmen ausgewählt aus Bewegung des Stents, Bewegung des Sprühdorns, Bewegung der Luftdüse, Regulierung des Sprühstrahls und Regulierung des Luftstroms, und
d) während der Durchführung der Schritte b) und c) ein Sprühstrahl der Sprühdüse auf den Gegenstrombereich auf die Außenseite des Stents gerichtet wird, wobei im Innern des Stents ein Luftstrom über die Luftdüse und ein Sprühstrahl über den Sprühdorn gleichzeitig erzeugt wird.

2. Verfahren nach Anspruch 1, bei dem luminal und abluminal mit unterschiedlichen Wirkstoffen beschichtet wird.

3. Verfahren nach Anspruch 2, bei dem luminal ein einheilungsfördernder Wirkstoff aufgetragen wird.

4. Verfahren nach Anspruch 3, bei dem der einheilungsfördernde Wirkstoff Atorvastatin ist.

5. Verfahren nach Anspruch 2, bei dem abluminal ein proliferationshemmender Wirkstoff aufgebracht wird.

6. Verfahren nach Anspruch 5, bei dem der proliferationshemmende Wirkstoff Sirolimus oder ein Derivat davon ist.

7. Verfahren nach einem der Ansprüche 1 bis 6, bei dem die Wirkstoffe in eine Trägermatrix eingebettet werden.

8. Verfahren nach Anspruch 7, bei dem die Trägermatrix biokorrodierbar ist.

9. Verfahren nach Anspruch 8, bei dem die Trägermatrix aus PLLA besteht.

## Claims

1. A method for coating a stent, comprising the following steps:
a) providing a device for coating a stent, which device comprises a holder for the stent, a spraying unit having a spray mandrel, and an air nozzle, wherein the spray mandrel, the air nozzle and the holder are designed and disposed relative to each other such that, during coating, the spray mandrel projects into the stent from one side and the air nozzle projects into the stent from the opposing side, and wherein a position of the stent relative to the spray mandrel and relative to the air nozzle can be varied during coating, or the stent, during coating, can be moved in the direction of the spray mandrel or in the direction of the air nozzle, and wherein
the spraying unit has a spray nozzle, which is directed at an abluminal side of the stent,
b) coating the luminal side of the stent by introducing a spray jet via the spray mandrel and an air flow via the air nozzle, in such a way that the spray jet is deflected radially outwardly by the air flow in the counterflow region; and
c) displacing a position of the counterflow region relative to the stent by at least one of the measures selected from moving the stent, moving the spray mandrel, moving the air nozzle, regulating the spray jet, and regulating the air flow, and
d) while carrying out steps b) and c), directing a spray jet of the spray nozzle at the counterflow region on the outside of the stent, wherein, inside the stent, an air flow is produced via the air nozzle and at the same time a spray jet is produced via the spray mandrel.

2. The method according to claim 1, in which the luminal and abluminal sides are coated with different active agents.

3. The method according to claim 2, in which a healing-promoting active agent is applied to the luminal side.

4. The method according to claim 3, in which the healing-promoting active agent is atorvastatin.

5. The method according to claim 2, in which a proliferation-inhibiting active agent is applied to the abluminal side.

6. The method according to claim 5, in which the proliferation-inhibiting active agent is sirolimus or a derivative thereof.

7. The method according to any one of claims 1 to 6, in which the active agents are embedded in a carrier matrix.

8. The method according to claim 7, in which the carrier matrix is biocorrodible.

9. The method according to claim 8, wherein the carrier matrix consists of PLLA.

## Revendications

1. Procédé destiné à revêtir une endoprothèse, comprenant les étapes de :
a) mise à disposition d'un dispositif destiné au revêtement de l'endoprothèse, qui comprend un support pour l'endoprothèse, un système de pulvérisation avec une pointe de pulvérisation et une buse à air, où la pointe de pulvérisation, la buse à air et le support sont conçus et disposés les uns par rapport aux autres de sorte que la pointe de pulvérisation rentre à l'intérieur de l'endoprothèse pendant le revêtement par un côté et la buse à air rentre à l'intérieur de l'endoprothèse par le côté opposé, et où, pendant le revêtement, la position de l'endoprothèse vis-à-vis de la pointe de pulvérisation et vis-à-vis de la buse à air peut être variée, ou
l'endoprothèse peut être déplacée pendant le revêtement en direction de la pointe de pulvérisation ou en direction de la buse à air, et où le système de pulvérisation présente une buse de pulvérisation qui est orientée sur un côté abluminal de l'endoprothèse,
b) revêtement luminal de l'endoprothèse par l'introduction d'un jet de pulvérisation par l'intermédiaire de la pointe de pulvérisation ainsi que d'un courant d'air par l'intermédiaire de la buse à air, de telle sorte que le jet de pulvérisation est dévié radialement vers l'extérieur par le courant d'air dans la zone à contre courant ; et
c) déplacement d'une position de la zone à contre courant par rapport à l'endoprothèse par au moins une des manoeuvres choisie parmi un déplacement de l'endoprothèse, un déplacement de la pointe de pulvérisation, un déplacement de la buse à air, un réglage du jet de pulvérisation et un réglage du courant d'air, et
d) pendant l'exécution des étapes b) et c), un jet de la buse de pulvérisation est orienté vers la zone de contre courant sur le côté externe de l'endoprothèse, où, à l'intérieur de l'endoprothèse, un courant d'air est généré par l'intermédiaire de la buse à air et un jet de pulvérisation est généré simultanément par l'intermédiaire de la pointe de pulvérisation.

2. Procédé selon la revendication 1, dans lequel les parties luminale et abluminale sont revêtues avec des matières actives différentes.

3. Procédé selon la revendication 2, dans lequel une matière active favorisant la guérison est introduite dans la partie luminale.

4. Procédé selon la revendication 3, dans lequel la matière active favorisant la guérison est de l'atorvastatine.

5. Procédé selon la revendication 2, dans lequel une matière active inhibitrice de prolifération est rapportée sur la partie abluminale.

6. Procédé selon la revendication 5, dans lequel la matière active inhibitrice de prolifération est du sirolimus ou un de ses dérivés.

7. Procédé selon l'une des revendications 1 à 6, dans lequel les matières actives sont incorporées dans une matrice support.

8. Procédé selon la revendication 7, dans lequel la matrice support est biocorrodable.

9. Procédé selon la revendication 8, dans lequel la matrice support est constituée d'acide poly L lactique PLLA.
